# EUROPEAN PATENT SPECIFICATION

(11) **EP 0 957 164 B1**
(45) Date of publication and mention of the grant of the patent: **21.03.2001**
(21) Application number: 98201633.9
(22) Date of filing: 15.05.1998
(51) Int. Cl.: C12N 15/12, C12N 15/62, G01N 33/68

(54) **Insulin binding polypeptide**
Insulinbindendes Polypeptid
Polypeptides liant l'insuline

(43) Date of publication of application: 17.11.1999
(73) Proprietor: NOVO NORDISK A/S, 2880 Bagsvaerd (DK)
(72) Inventor: Kristensen, Claus, 1876 Frederiksberg C (DK)
(74) Representative: Hoeiberg, Susanne

(56) References cited:
- WO-A-91/17252
- MCKERN N M ET AL: "Crystallization of the first three domains of the human insulin-like growth factor-1 receptor" PROTEIN SCIENCE, vol. 6, no. 12, December 1997, pages 2663-2666, XP002081790
- WAUGH S M ET AL: "Isolation of a proteolytically derived domain of the insulin receptor containing the major site of cross-linking/binding" BIOCHEMISTRY, vol. 28, 1989, pages 3448-3455, XP002081791
- KRISTENSEN C ET AL: "Expression and characterization of a 70-kDa fragment of the insulin receptor that binds insulin" JOURNAL OF BIOLOGICAL CHEMISTRY, vol. 273, no. 28, 10 July 1998, pages 17780-17786, XP002081792

## Description

### Technical field

The present invention relates to the construction of a polypeptide capable of binding insulin and/or insulin-like growth factors and insulin mimetics, having a molecular weight between 40 kD and 100 kD and the use of a polypeptide for the screening of ligands binding to the polypeptide.

### Background of the invention

Insulin mediates its effects by binding to specific tyrosine kinase receptors in the plasma membrane of target cells. The structure of the insulin receptor has been investigated extensively and recently reviewed (Lee & Pilch, Am. Journ. Physiology, 1994, **266**, pp. 319-334; Tavaré & Siddle, Biochem. Biophys. Acta, 1993, **1178,** pp. 21-39). A number of naturally occurring mutations in the insulin receptor gene that effects receptor function have been identified and reviewed by Taylor et al., Endocrine reviews, 1992, **13**, pp. 566-595 and Endocrine reviews, 1994, **2**, pp. 58-65.

The insulin receptor is a glycoprotein of a relative molecular mass of 350-400 kDa, which is synthesised as a single chain polypeptide and proteolytically cleaved yielding a disulphide linked α-β monomer insulin receptor. Two α-β monomers are linked by disulphide bonds between the α-subunits, resulting in the β-α-α-β receptor subunit configuration. The α-subunit is 135 kDa and the β-subunit is 95 kDa.

The intracellular part of the β-subunit includes the tyrosine kinase domain which acquire kinase activity upon binding of insulin to epitopes in the ectodomain. The X-ray crystal structure of the tyrosine kinase domain has been solved (Hubbard et al., Nature, 1994, **372**, pp. 746-754) whereas detailed three-dimensional structure of the insulin binding site is not available.

On alignment with epidermal growth factor receptor sequences the consensus is that the insulin receptor α-subunit has two large domains L1 and L2 separated by a cysteine-rich region. The L1 and L2 domains are predicted to be comprised of four repeats of α-helices followed by β-strand, turn and β-strand. The best conserved feature in these repeats is a central glycine residue responsible for the turn (Bajaj et al., Biochem. Biophys. Acta, 1987, **916,** pp. 220-226). The L1 region spans amino acids 1-155. The cysteine rich region comprises residues 155-312 and L2 comprises residues 313-468 (Ward et al., Proteins: Structure, Function and Genetics, 1995, **22**, pp. 141-153).

Recently McKem et al., Protein Science, 1997, **6**, pp. 2663-2666, described the crystallisation of the first three domains of the human insulin-like growth factor-1 receptor. The 63 kDa fragment comprised amino acids 1-462 of the L1-cystein-rich L2 region of the ectodomain but was unable to bind insulin-like growth factor.

Insulin receptor deletion constructs have previously been expressed by Sung et al., Molecular Endocrinology, 1994, **8,** pp.315-324, who expressed a receptor with deletion of the residues 485-599 from the α-subunit having a size of 127 kDa. They found that that the mutated receptor bound insulin with high-affinity. Zhang and Roth, Proc. Natl. Acad. Sci., USA, 1991, **88**, pp. 9858-9862, studies indicate that residues 450-601 constitute a region of high-affinity binding of insulin.

The major ligand binding determinants of the IR appear to reside in the α-subunit, more precisely in the N-terminal region, L1 (Mynarcik et al., Jour. Biol. Chem., 1997, **272**, pp. 18650-18655).

The insulin contact sites are apparently exclusively located in the α-subunit, Schaefer et al., Jour. Biol. Chem., 1990, **265**, pp. 13248-13253 examined the expression of free α-subunit. Schaefer et al. describe a series of truncations of the IR including a free α-subunit and IR1-486, the latter is essentially the first three domains of IR. The α-subunit was secreted as a monomer that bound insulin with near wild-type affinity, but the expression level of free α-subunit was low allowing only limited characterisation. The results point to the fact that all constructs in which the carboxy end of the α-subunit is removed fail to bind insulin, which points to the fact that in addition to the first three domains of the IR a fourth domain from the carboxy end of the α-subunit is necessary for binding.

For a receptor to bind insulin with the same affinity as the wild type receptor several crucial hormone-receptor contact sites (regions) have been identified, some of which are mentioned above.

Mynarcik et al., Jour. Biol. Chem., 1996, **271**, 2439-2442, performed alanine scanning studies that suggests that amino acids 704-717 of the C terminus of the insulin receptor represent a contact site for insulin. Of the 14 residues in this region only 4 amino acids, Asp-707, Val-712, Pro-716 and Arg-717 could be mutated to alanine without compromising insulin binding.

Kurose et al., Jour. Biol. Chem., **1994**, 269, pp. 29190-29197, who performed affinity labelling describe a site within the insulin receptor ectodomain which forms a binding pocket for B25 Phe. The fragment identified comprised residues 704-718 of the COOH terminus of the α-subunit. The results points in the direction of a binding region being located in the α-subunit upstream from exon 11 and consisting of 12-amino acids extending from Thr⁷⁰⁴- Lys⁷¹⁸.

The prior art examinations of insulin binding sites have revealed specific amino acid regions necessary for high-affinity insulin binding. In order to examine the structure-activity relationship of a ligand-receptor complex the fragments may be crystallised. However, the fragments synthesised have been of a size of no less than 127 kD, which has resulted in unsuccessful crystallisation. This may possibly be due to their sizes.

The general approach when searching for the physical and chemical properties of ligand-receptor complexes is: given a ligand-receptor complex, the ligand is first analysed to isolate the functional groups critical for recognition and binding. These are the pharmacophoric elements. The analysis of the structure-activity relationship can be examined by co-crystallisation of the ligand-receptor complex combined with binding studies. The pharmacophoric elements are relevant when developing pharmaceuticals.

### Summary of the invention

Accordingly the present invention relates to a polypeptide having binding affinity for insulin and/or insulin-like growth factor, wherein the molecular weight of the polypeptide is between 40 kD and 100 kD.

In a related aspect the present invention provides successful identification and characterisation of a polypeptide corresponding to a small fragment of the insulin receptor. This receptor fragment may be a free monomeric α-subunit deleted of amino acids 469-703 and 718-729 or a functional equivalent thereof, binding insulin with an affinity below 10⁻⁸ as measured in a polyethylene glycol (PEG) precipitation assay and having the formula

NH₂-A-B-COOH

wherein A is an amino acid sequence comprising a sequence corresponding to SEQ ID NO: 1 , SEQ ID NO: 2 or SEQ ID NO: 3 or a functional equivalent thereof
and B is an amino acid sequence comprising a sequence corresponding to SEQ ID NO: 4, SEQ ID NO: 5, SEQ ID NO: 6, SEQ ID NO: 7, SEQ ID NO: 8, SEQ ID NO: 9, SEQ ID NO: 10 or SEQ ID NO: 11 or a functional equivalent thereof.

Due to the small size of the polypeptide it is possible to co-crystallise it with insulin, obtaining the three-dimensional structure of the binding fragment. It was a surprise for the inventors that it is possible to construct a polypeptide of that small a size without impairing the binding affinity compared to the binding affinity of the soluble wild-type receptor.

Having obtained the three-dimensional structure of the binding fragment it is possible to screen for insulin mimetics, i.e. ligands binding to the insulin receptor exerting insulin activity.

Accordingly an aspect of the present invention is the use of the polypeptide for the screening of ligands binding to the polypeptide.

Another aspect of the present invention is an expression cassette comprising a DNA fragment encoding a polypeptide according to the invention, including a fusion polypeptide construct.

Yet another aspect of the present invention is a fusion polypeptide comprising the polypeptide and another polypeptide, wherein said other polypeptide is capable of facilitating the expression, purification and/or crystallisation of the polypeptide. The fusion polypeptide may comprise antigenic peptide -Tag and/or FLAG epitop, or it may comprise polyHis and/or 6His Tag.

### Figures

The following is a short description of the figures illustrating the invention.

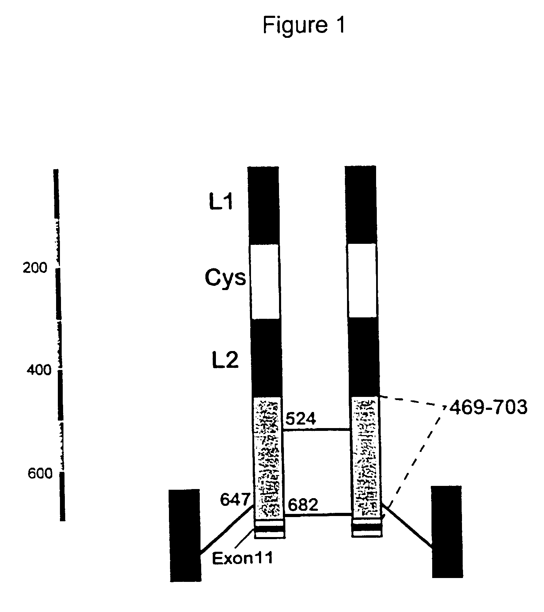

Figure 1. shows a diagram of the major structural features of the insulin receptor α-subunit. Shown is the insulin receptor ectodomain, the diagram illustrates major domains, such as a polypeptide corresponding to L1 and L2; cysteine rich domain (Cys); the alternative spliced region encoded by exon11 (Exon11).

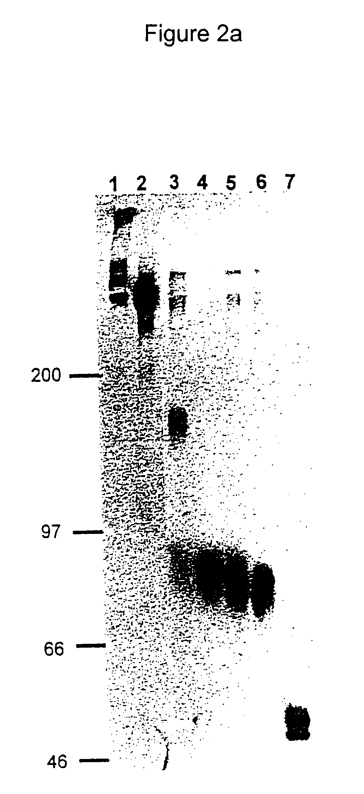

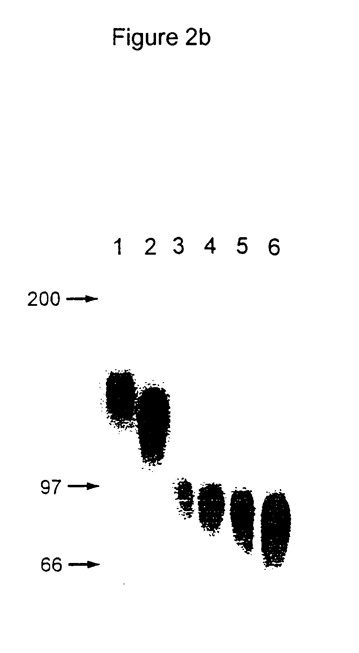

Figure 2a & b show the immunoblotting of receptor constructs secreted from BHK cells. Medium from BHK cells transfected with receptor deletion constructs was collected. 15 µl of medium was mixed with 5 µl of SDS loading buffer and proteins were separated on 6% SDS polyacrylamide gel and blotted onto PVDF membrane. IR α-subunit was detected using a monoclonal antibody specific for epitope near N-terminal of α-subunit. The following samples were analysed: IRwt *(lane 1),* IRΔ599 *(lane 2),* IRΔ673 *(lane 3)*, IRΔ685 *(lane 4)*, IRΔ685* *(lane 5)*, IRΔ703 *(lane* 6), and IRΔ703 treated with PNGase F *(lane 7*, Fig. 2a only). Samples in Fig. 2a were unreduced and in Fig. 2b samples were reduced by adding DTT and boiling before loading onto gel. Molecular mass markers (Rainbow from Amersham) are indicated to the left.

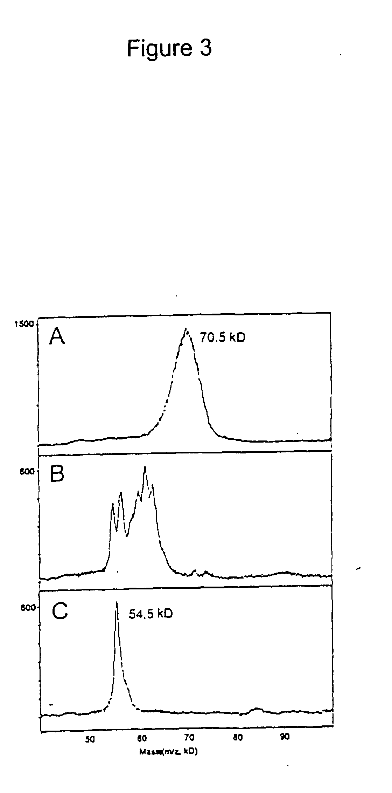

Figure 3 shows a MALDI-TOF mass spectra of IRΔ703. Mass spectra of native IRΔ703 protein (Panel A) or IRΔ703 treated with a deglycosylation cocktail containing neuraminidase, EndoH, and PNGaseF. Complete deglycosylation was obtained in the presence of 0.1% SDS (Panel C). In the absence of SDS only partial deglycosylation was obtained (Panel B).

BHK, baby hamster kidney; BSA, bovine serum albumin; GH, growth hormone; IgG, ImmunoglobulinG; IGFI, insulin-like growth factor I; IR, insulin receptor; MALDI-TOF, matrix associated laser desorption ionization-time of flight; PCR, polymerase chain reaction; SDS, sodium dodecyl sulphate.

### Detailed description of the invention

An object of the present invention is a polypeptide having a binding affinity for insulin and/or insulin-like growth factor, where the molecular weight of the polypeptide is between 40 kD and100 kD. The polypeptide according to the present invention may, due to its size, be co-crystallised with insulin and thereby make it possible to obtain knowledge of the structure-activity relationship. It is of importance that the polypeptide according to the invention is not too small in order to be able to mimic the original insulin receptor.

Accordingly, the molecular weight of the polypeptide is preferably above 45 kD, more preferably above 50 kD.

To ensure an improved crystallisation process the polypeptide is preferably below 90 kD, more preferably below 80 kD. The molecular weights indicated relate to the reduced form of polypeptide purified from mammalian cell culture medium (e.g. BHK cells). The purified polypeptide has not been modified with enzymes or chemicals, and has retained the ability to bind insulin. The molecular weight may be measured by the MALDI-TOF method.

The polypeptide of present invention is of the formula

NH₂ - A - B - COOH

wherein A is an amino acid sequence comprising a sequence corresponding to SEQ ID NO: 1 , SEQ ID NO: 2 or SEQ ID NO: 3 or a functional equivalent thereof and B is an amino acid sequence comprising a sequence corresponding to SEQ ID NO: 4, SEQ ID NO: 5, SEQ ID NO: 6, SEQ ID NO: 7, SEQ ID NO: 8, SEQ ID NO: 9, SEQ ID NO: or SEQ ID NO: 11 or a functional equivalent thereof, and has a binding affinity below 10⁻⁸ as measured in a polyethylene glycol (PEG) precipitation assay.

The sequences of A may individually be combined with the sequences of B or their functional equivalents.

In a preferred aspect of the invention A comprises a sequence corresponding to SEQ ID NO: 1, SEQ ID NO:3 or a functional equivalent thereof and B comprises a sequence corresponding to SEQ ID NO: 4, SEQ ID NO: 5, SEQ ID NO: 10 or a functional equivalent thereof.

SEQ ID NO: 1 corresponds to the main binding domains of the insulin receptor, e.g. domains L1 - CYS - L2 that have been acknowledged as being of importance for binding insulin. The polypeptide may comprise other amino acid sequences as long as they are not impairing the binding function of the sequence. SEQ ID NO: 2 corresponds to the equivalent domain on the insulin-like growth factor receptor (IGF-IR).

By the term a functional equivalent is meant a sequence which process a corresponding binding ability for insulin as SEQ ID NO: 1 but wherein one or more amino acids have been substituted with others. Preferably a functional equivalent contains conservative substitutions, i.e. in which one or more amino acids are substituted for another amino acid that has similar properties, such as one skilled in the art of protein chemistry will expect a secondary structure in hydropatic nature of the polypeptide to be substantially unchanged. Amino acids suitable for conservative substitutions include those having functionally similar side chains. For example, hydrophobic residues: *e.g.* glycine, alanine, valine, leucine, isoleucine and methionine may replace another such residue. Similarly, conservative substitutions may involve interchanging hydrophilic residues: (*e.g.*: arginine and lysine, glutamine and aspargine, threonine and serine), basic reduces (*e.g*., lysine, arginine and histidine), and/or acidic residues (*e.g.*, aspartic acid and glutamic acid). Functional equivalents may also, or alternatively, be modified by for example the deletion or addition of amino acids, or the chemical modification of amino acids, that have minimum influence on the binding abilities of the polypeptide. By performing deletions and/or additions it is of importance that the molecular weight of the polypeptide is within the limits specified above.

SEQ ID NO: 3 corresponds to a chimeric polypeptide of SEQ ID NO: 1 and SEQ ID NO: 2 comprising the IGFIR sequence with the first 62 amino acids replaced by corresponding 68 amino acids of IR.

The chimeric polypeptide may be constructed by any combination of the two receptors, provided the polypeptide retains its binding affinity. A chimeric polypeptide may also be constructed by combinations of functional equivalents to either or both of the receptors represented by SEQ ID NO: 1 and SEQ ID NO: 2. By the construction of the chimeric polypeptide it is possible to tailor the polypeptide for crystallisation.

SEQ ID NO: 4-11 correspond to C-terminal parts of the α-subunit of the wild type insulin receptor or insulin-like growth factor receptor.

Preferred are the smallest possible polypeptide constructs capable of providing the polypeptide with insulin binding activity, i.e. constructs comprising as B SEQ ID NO: 4, 5 and 10.

SEQ ID NO: 4 corresponds to SEQ ID NO: 5 apart from 12 amino acids corresponding to the so-called exon 11 of the insulin receptor.

With respect to B, chimeric constructions between the insulin receptor and IGF-IR or their functional equivalent are within the scope of the invention.

The binding affinity of the polypeptide according to the invention is surprisingly comparable to the binding affinity of the soluble insulin receptor ectodomain, and close to the binding affinity of the wild-type insulin receptor. Binding affinity may be measured by the polyethylene glycol (PEG) precipitation assay as described in the examples below.

As measured with the polyethylene glycol precipitation assay the binding affinity of the polypeptide according to the invention is below 10⁻⁸, more preferably below 10⁻⁹ M.

The polypeptide according to the invention may structurally mimic the original insulin receptor, thereby being capable of offering knowledge of the three-dimensional structure of the insulin binding sites of the insulin receptor.

The polypeptide having binding affinity for insulin and/or insulin like growth factor may be crystallised to obtain the atomic structures of the insulin binding regions of the insulin receptor. Preferably the smaller and more homogenous the molecule is the better the outcome of the crystallisation will be.

The preferred polypeptide has a mass found by mass spectrometry of approximately 70 kD. This small insulin receptor fragment bound insulin with an affinity (K_{d}) of 4.4 nM, which is similar to what was found for the full-length soluble ectodomain of the insulin receptor (5.0 nM). Cross-linking experiments may confirm that the polypeptides specifically bind insulin and/or insulin-like growth factors.

To provide homogeneous polypeptide compositions it is important to control the amount of posttranslational modifications of the polypeptide. Modifications such as glycosylation of the polypeptide are of importance. In a preferred embodiment the polypeptide according to the invention has at most 7 N-linked glycosylation sites, more preferably at most 6 N-linked glycosylation sites. In order to minimise the amounts of glycosylation sites the polypeptide may be subjected to modifications wherein the glycosylation motive, Asn-x-Ser/Thr is modified by substitution of any of Asn, Ser, Thr by any other amino acid residue, such as by substituting Asn with Gln.

In another embodiment the polypeptide may be subjected to enzyme treatment after purification in order to remove all or some of the glycosylation before crystallisation.

Another object of the invention is a fusion polypeptide comprising a polypeptide as defined above fused to another peptide(s). The latter may serve at least one of several purposes, such as: facilitating expression of the polypeptide thereby increasing the yield of expressed polypeptide.

Another purpose may be to ease the purification of the polypeptide, and a third purpose may be to facilitate the crystallisation of the polypeptide.

Depending on the purpose of the fusion peptide it may contain a cleavage site, whereby the polypeptide according to the invention may be cleaved off the other peptide(s).

The fusion peptide construct according to the invention may consist of the IR ectodomain fused to the Fc region of the IgG heavy chain and, may also (or instead) consist of any suitable peptide, such as polyHis, 6His Tag, antigenic peptide-Tag, FLAG epitope (an antigenic peptide tag) and/or the β-subunit of the insulin receptor or fragments thereof.

A preferred fusion peptide comprises the polypeptide according to the invention, a tetrabasic cleavage site, the extracellular part of the β-subunit, and the Fc domain from IgG.

Another object of the invention is a DNA fragment encoding the polypeptide, or the fusion polypeptide as defined above.

A further object of the invention is an expression cassette comprising a DNA fragment as described above, as well as a cell capable of expressing a polypeptide being transformed with the expression cassette.

Furthermore the invention relates to a process of producing a polypeptide, wherein a cell containing a recombinant expression vector comprising a DNA construct encoding a polypeptide, is cultured in a suitable medium under conditions which promote the expression of the polypeptide, and where the polypeptide is recovered from the culture.

The polypeptide according to the invention is particularly useful for determining the three-dimensional structure of the insulin binding sites of the original insulin receptor.

Accordingly, the polypeptide may be used for determining the structural demands for insulin mimetics, and the polypeptide may thus be used for screening of ligands binding to the polypeptide, and thereby be used for the screening for insulin mimetics.

Yet another object of the present invention is an insulin mimetic having insulin activity when binding to an insulin receptor in vivo, and being capable of binding to the polypeptide according to the invention.

By the term insulin mimetic is meant a chemical and/or biochemical compound, excluding insulin, having insulin activity. In a preferred embodiment the insulin mimetic is an oral insulin mimetic, i.e. a chemical compound useful for being formulated into tablets and/or other oral medicaments.

### EXAMPLES

The following is a description of the experiments performed to express and examine the insulin binding polypeptide.

### Materials and methods

Experiments were performed using insulin and A14-¹²⁵I-insulin and A14-¹²⁵I. Both from Novo Nordisk.

cDNA encoding IR-IgG fusion protein was a gift from Dr. Joseph Bass, University of Chicago. The DNA restriction enzymes: T4 ligase, EndoH, and PNGaseF were from New England Biolabs, Pwo polymerase was from Boehringer Mannheim. Neuraminidase was from Sigma Chemical Co.

The preparation of plasmid DNA, agarose and polyacrylamide gel electrophoresis were performed according to standard methods. For DNA minipreps QIAprep 8 kit was used (QIAGEN). BHK (baby hamster kidney) cells were grown in Dulbecco's modified Eagle's medium (Life Technologies Inc.) supplemented with 10% or 2% fetal calf serum.

### Example 1

### Construction of cDNA Expression Plasmids encoding the polypeptide Receptor Constructs

According to the invention the polypeptides in Table 1 and, as comparison, wild type α-subunit, and the polypeptide of Sung et al. described above were constructed as receptor constructs.

Table 1 shows an overview of the polypeptide receptor constructs.

**Table 1**

| Construct ¹⁾ | Corresponding IRwt deleted of amino acids | A comprising (SEQ ID NO) | B comprising (SEQ ID NO) | Reduced molecular weight (approx. kD estimated from SDS-PAGE) |
|---|---|---|---|---|
| IRwt ²⁾ | | | | 137 |
| lRΔ599 ³⁾ | 487-599 | 1 | - | 127 |
| IRΔ673 | 469-673 | 1 | 8 | 97 |
| IRΔ685 | 469-685 | 1 | 6 | 90 |
| IRΔ685* | 469-685,718-729 | 1 | 7 | 85 |
| IRΔ703 | 469-703,718-729 | 1 | 5 | 80 |
| IRΔ703* ⁴⁾ | 469-703,718-729 | 1 | 5 | 80 |
| IGFIR(Δ459-672).1-68IR ⁵⁾ | 459-672, 1-68 | 3 | 11 | 80 |
| IGFIR(Δ459-672).1-68, | 459-672,1-68, | 3 | 5 | 80 |
| 704-729.IR ⁵⁾ | 704-729 | | | |
| sIR | | | | 137 |

| | | | | |
|---|---|---|---|---|
| 1) sIR is the soluble IR ectodomain, all other constructs are expressed as IR ectodomain fused to the Fc region of IgG. Δ denotes deletion and the number is the last amino acid deleted. IRΔ685*, IRΔ703 and IRΔ703* are exon11- others are exon11+. | | | | |
| 2) Soluble IR ectodomain fused to Fc from IgG C-terminally. | | | | |
| 3) For comparison is the polypeptide disclosed by Sung et al. described above. | | | | |
| 4) Expressed having a stop codon immediately after B, not expressed as a fusion peptide. | | | | |
| 5) Constructs were expressed as IGFIR ectodomain fused to the Fc region of IgG. | | | | |

The receptor constructs were expressed as soluble insulin receptor (IR) IgG fusion protein, consisting of the IR ectodomain fused to the Fc region of the IgG heavy chain. The IR-IgG fusion constructs were expressed in baby hamster kidney (BHK) cells by inserting the sequence encoding the fusion polypeptide into the ZEM expression vector and transfecting this into BHK cells as described previously.

### Receptor construct IRΔ599

The Receptor construct IRΔ599 was made by PCR amplification using the sense primer 5'-CCTCTAAGATCTTGGATCCAATCTCAGTG-3' (Bglll and BamHI sites underlined) and an antisense primer downstream from EagI site (amino acid 678-680). This fragment was digested with Bglll and EagI and ligated into corresponding site of the plasmid encoding IRwt, resulting in a cDNA sequence in which amino acids 485-599 are deleted compared to the wildtype α-subunit.

### Receptor construct IRΔ673

IRΔ673 was constructed by overlap extension of fragments I and II.

Fragment I was amplified using a sense primer upstream from Bsu361 site and the antisense primer 5'-CTCACAGCTAGCCTTGTCCCCATTGGTC-3' (Nhel site underlined).

Fragment II was amplified using sense primer 5'-GGGGACAAGGCTAGCTGTGAGTATGAGGATTCGGCCGGCG-3' (Nhel site underlined) and an antisense primer downstream from Avrll site (amino acids 728-729 in exon11). The overlap fragment was digested with Bsu361 and AvrII and ligated into corresponding site of the plasmid encoding IRwt, resulting in a cDNA sequence deleted of amino acids 469-673 compared to the wildtype α-subunit, and a new silent Nhel site is introduced (amino acids 466-467).

### Receptor construct IRΔ685

IRΔ685 was made using the sense primer 5'-GACAAGGCTAGCTGTCCAAAGACAGACTCTCAGATCC -3' and an antisense primer downstream from Avrll site (amino acids 728-729). This fragment was digested with NheI and Avrll and ligated into a corresponding site of the plasmid encoding IRΔ673, resulting in a cDNA sequence deleted of amino acids 469-685 compared to the wildtype α-subunit.

### Receptor constructs IRΔ685* and IRΔ703

Finally IRΔ685* and IRΔ703 were made by PCR amplification using a sense primer upstream from NarI site (amino acids 450-452) and the antisense primers 5'-TTTTCCTAGGGACGAAAACCACG -3' and 5'- TTTTCCTAGGGACGAAAAC-CACGTTGTGCAGGTAATCCTCAAACGTACAGCTAGCCTTGTCCCC (AvrII site underlined). The fragments were digested with Narl and Avrll and ligated into corresponding site of the plasmid encoding IRwt, resulting in cDNA sequences deleted of amino acids 469-685+718-729 and 469-703+718-729 respectively compared to the wildtype α-subunit. In these two constructs a new silent Avrll site was introduced (amino acids 716-717),

### Receptor construct IRΔ703*

A stopcodon was inserted in the DNA sequence after codon for amino acid **Serine of SEQ ID NO: 5** by PCR amplifying the IRΔ703.ZEM vector with antisense oligonucleotide 5'-TTTTTCTAGACTAAGATGGCCTAGGGACG-3' (Xbal site underlined) and an sense primer upstream from Bsu36l site. The PCR fragment was digested with Bsu36I and Xbal and ligated into corresponding site of plasmid encoding IRwt.

### Receptor constructs IGFIR(Δ459-672).1-68IR and IGFIR(Δ459-672).1 -68, 704-729IR

First a construct encoding IGFIR deleted of the amino acids 459-672 was made by overlap extension of fragments III and IV.

Fragment III was amplified with IGFIR sequence as template using a sense primer upstream from Narl site (aminoacids 440-441) and the antisense primer 5'- GGGACAGCTAGCTCTCTCCCCGTTCGTTCCTGG- 3' (Nhel site underlined).

Fragment IV was amplified with IGFIR sequence as template using a the sense primer 5'- GAGAGCTAGCTGTCCCAAAACTGAAGCCGAG - 3' (Nhel site underlined) and an antisense primer downstream from EagI site (amino acids 728-730).

The overlap fragment was digested with Narl and EagI and ligated into corresponding site of the plasmid encoding IGFIR ectodomain fused to Fc domain from IgG. This results in a cDNA sequence (IGFIR(Δ459-672)) deleted of aminoacids 459-672 compared to wildtype IGFIR α-subunit, and a new silent Nhel site is introduced (amino acids 456-457).

IGFIR(Δ459-672).1-681R was now made by inserting BssHII/XbaI fragment from IGFIR(Δ459-672) into vector encoding IGFIR.1-68IR. IGFIR.1-68IR is IGFIR with the first 62 amino acids replaced by homologous 68 amino acids from IR, a construct that was obtained by using Xho site that is similarly positioned in IR and IGFIR.

IGFIR(Δ459-672).1-68, 704-7291R was made by replacing NheI/XbaI fragment of IGFIR(Δ459-672).1-68IR with Nhel/Xbal fragment from IRΔ703.

The constructs produced were used for binding studies as described below.

### Example 2

### Receptor Constructs: Binding of Insulin

Insulin receptor secreted into BHK medium was analysed in the binding assay polyethylene glycol (PEG) precipitation assay.

In the assay the concentration of receptor was adjusted to yield 10-15% binding of tracer when no competing ligand was added to the competition assay. The binding data were fitted using non-linear regression algorithm in GraphPad Prism 2.01 (GraphPad Software Inc, San Diego, CA).

Each affinity in Table 2 is the average for at least three independent experiments. The data were determined from binding curves.

### PEG assay

The precipitation assay was performed by incubating a suitable dilution of BHK medium containing: receptor in a total volume of 200 µI with A14-¹²⁵I-insulin (5-10 pM) and varying concentrations of unlabeled ligand in a binding buffer for 16 hours at 4°C. The binding buffer consisted of 100 mM Hepes (pH 8.0), 100 mM NaCI, 10 mM MgCl₂, 0.05 % (w/v) BSA, 0.025 % (W/v) Triton X-100. Subsequently, bound counts were recovered by precipitation with 0.2% γ-globulin and 500 µl of 25% (w/v) polyethylene glycol 8000. Bound A14-¹²⁵I-insuIin were counted in a γ-counter.

Table 2 shows the insulin affinities (K_{d}) of the receptor constructs.

**Table 2**

| Construct ¹⁾ | Affinity (K_{d}) PEG assay (nM) |
|---|---|
| Irwt | 3.1 ²⁾ |
| IRΔ599 | 2.3 |
| IRΔ673 | 1.1 |
| IRΔ685 | 6.4 |
| IRΔ685* | 7.3 |
| IRΔ703 | 4.4 |
| IRΔ703* | 3.9 |
| IGFIR(Δ459-672).1-68IR | 2.1 |
| IGFIR(Δ459-672).1-68, 704-729.IR | 4.4 |
| SIR | 5.0 |

| | |
|---|---|
| ¹⁾ sIR is soluble IR ectodomain, all other constructs are expressed as IR ectodomain fused to Fc region of IgG. Δ denotes deletion and the number is the last amino acid deleted. IRΔ685*, IRΔ703 and IRΔ703* are exon11- others are exon11+. | |
| ²⁾ Affinities of IRwt are for low affinity site only. | |

The PEG assay showed binding affinities for all receptors in the nM range, which is similar to what is found for the full-length ectodomain. The lowest K_{d} of 1.1 nM was observed for IRΔ673, and the shortest construct IRΔ703 gave a K_{d} of 4.4 ± 0.8 nM. For the full-length ectodomain the affinity for insulin was 5.0 ± 0.2 nM.

Two of the deletion constructs IRΔ685 and IRΔ685* only differ in the exon11 region (amino acids 718-729); the affinity of IRΔ685 for insulin was 6.4 ± 2.4 nM and when exon11 is deleted in IRΔ685* the affinity was 7.3 ± 1.6 nM. In these receptors the exon11 region does not influence binding of insulin significantly.

Thus, according to the invention it has been demonstrated, that it is possible to construct a very small insulin receptor polypeptide comparable to the wild type α-subunit deleted of 247 amino acids, without compromising insulin binding.

### Example 3

### Immunoblotting

The expressed receptors were detected by immunoblotting using the monoclonal antibody mAb-F26. This antibody was raised against a peptide corresponding to amino acids 39-75, mapping at the amino terminus of the insulin receptor α-subunit. The antibody was kindly donated by Jes Thorn Clausen, Novo Nordisk.

To perform the immunoblotting, medium from BHK cells expressing receptor constructs was mixed with 0.33 volume of SDS-PAGE loading buffer (40% w/v Sucrose, 563 mM Tris Base, 423 mM Tris HCI, 278 mM SDS, 2 mM EDTA, 0.88 mM Serva Blue G250, 0.7 mM Phenol Red). 10 µl was run on a 6% SDS-polyacrylamide gel. Reduced samples were mixed with loading buffer containing 0.1 M dithiotreithol (DTT) and incubated at 70 °C for 10 min before loading 10 µl on SDS-membrane (Millipore). The membrane was blocked by incubating with blocking buffer (5% defatted skim milk, 2% BSA in TBS (150 mM NaCI, 10 mM Tris, HCI pH 7.5) for 16 hours at 4°C. The receptor antibody mAb-F26 was diluted in blocking buffer and after incubating with receptor antibody the membrane was washed with TBS before incubating with peroxidase conjugated rabbit anti mouse immunoglobins antibody (P260 from DAKO, Denmark). Finally the blot was washed with TBS and immunoreactive protein was detected using an ECL reagent from Amersham.

### Detecting Receptor Fragments by Immunoblotting

The antibody used for immunoblotting was raised against an amino terminal epitope of the insulin receptor, and thus the antibody would be expected to recognise the α-subunit of all receptor deletion constructs expressed. Immunoblotting was performed on non-reduced as well as reduced samples of medium from transfected BHK cells.

For immunoblotting similar volumes of BHK medium was loaded, so in addition to smaller size there seems to be better level of expression when expressing the smaller receptor fragments.

The immunoblots are shown in Fig. 2a and Fig 2b.

On the reduced gel (Fig. 2b) the antibody detects the full-length α-subunit of 130 kD in the IRwt sample, whereas the constructs show a gradual decrease in size of the α-subunit from apparent MW of 130 kD to approximately 80 kD for the smallest receptor construct IRΔ703 (lane 6). This receptor is also shown in its deglycosylated form after PNGaseF treatment, where it has an apparent MW of approximately 55 kD (lane 7) (Fig. 2a).

Immunoblotting of the unreduced samples reveals three groups of immunoreactive bands (Fig. 2a). The full-length receptor fusion IRwt has been reported to migrate as 380 kD protein on SDS-PAGE consistent with the high molecular weight band of more than 200 kD observed on the blot (Fig. 2A).

There seem to be small amounts of monomeric α-subunit in the IRΔ673 construct probably because the α-α dimer is not very stable when one of the α-α disulphides (Cys524) has been removed. Possibly interactions between the Fc regions fused to the ectodomain also stabilise the disulphide bridge between the two α-subunits.

Finally the constructs IRΔ685 (lane 4), IRΔ685* (lane 5) and IRΔ703 (lane 6) all represent free monomeric α-subunit fragments only.

### Example 4

### Deglycosylation and Mass Spectrometry

The smallest polypeptide receptor fragment, IRΔ703 was purified by affinity chromatography using immobilised insulin. IRΔ703 in Hepes pH 8.0, 0.5% n-octylglucopyranoside, was treated with a deglycosylation cocktail containing neuraminidase, EndoH, and PNGaseF, either in the presence or absence of 0.1% SDS, for 18 hours at 37 °C. MALDI-TOF mass spectra were recorded on a Voyager DE (Perseptive Biosystems) in sinapinic acid matrix. Calibration was performed on the MH⁺ and MH₂²⁺ ions of human serum albumin.

The mass of IRΔ703 was found by mass spectrometry to be 70.5 kD (Fig. 3, panel A) which is somewhat lower than the apparent MW observed by SDS-PAGE (Fig. 2a & b). The band was fairly broad, presumably due to glycosylation heterogeneity. Complete deglycosylation in the presence of 0.1% SDS gave a sharper peak at 54.5 kD (Fig. 3, panel C) which is corresponding to the calculated value of 55.2 kD. Partial deglycosylation gave a series of peaks with a spacing of 1.5-2.0 kD corresponding to the core protein with 0,1, 2, 3, 4 and 5 remaining carbohydrate chains (Fig. 3, panel B). The difference in MW between the native and the completely deglycosylated forms is consistent with extensive use of the potential N-glycosylation sites.

### Example 5

### Crystallisation

The identification of a minimal ligand-receptor complex has facilitated the generation of crystals suitable for detailed structural analysis by X-ray crystallography.

The minimised insulin receptor may be co-crystallised with a ligand (insulin, insulin analogue or IGFI).

Crystals may be grown by the hanging drop vapor diffusion method using purified neuramidase-treated protein concentrated in Centricon 10 concentrators (Amicon Inc., USA) to 10-20 mg/ml. The receptor was mixed with one equivalent of ligand (insulin, insulin analogue or IGFI) and crystallised by equilibration with 11 % polyethylene glycol 6000 and 0.1 M imidazol-HCI pH 6.4. Using these conditions cubic crystals of 0.03* 0.03 * 0.5 mm grew within one week.

## Claims

1. A polypeptide having binding affinity for insulin, wherein the molecular weight of the polypeptide is between 40 kD and 100 kD, having the formula
NH₂ - A - B - COOH
wherein A is an amino acid sequence comprising a sequence corresponding to SEQ ID NO: 1 , SEQ ID NO: 2 or SEQ ID NO: 3 or a functional equivalent thereof
and B is an amino acid sequence comprising a sequence corresponding to SEQ ID NO: 4, SEQ ID NO: 5, SEQ ID NO: 6, SEQ ID NO: 7, SEQ ID NO: 8, SEQ ID NO: 9, SEQ ID NO: 10 or SEQ ID NO: 11 or a functional equivalent thereof, and
**wherein the binding affinity is below 10**^{**-8**} **as measured in a polyethylene glycol (PEG) precipitation assay.**

2. A polypeptide according to claim 1, wherein A comprises a sequence corresponding to SEQ ID NO: 1, SEQ ID NO:3 or a functional equivalent thereof
and B comprises a sequence corresponding to SEQ ID NO: 4, SEQ ID NO: 5, SEQ ID NO: 10 or a functional equivalent thereof.

3. A polypeptide according to any of the preceding claims, wherein the molecular weight of the polypeptide is below 90 kDa, preferably below 80 kDa.

4. A polypeptide according to any of the preceding claims, having at most 7, preferably at most 6 N-linked glycosylations sites.

5. A DNA fragment encoding a polypeptide as defined in any of the claims 1-4.

6. A fusion polypeptide comprising a polypeptide as defined in any of the claims 1-5, and another polypeptide, wherein said other polypeptide is capable of facilitating expression, purification and/or crystallisation of the polypeptide.

7. A fusion polypeptide according to claim 6, comprising antigenic peptide-Tag and/or FLAG epitop.

8. A fusion polypeptide according to claim 6, comprising polyHis and/or 6His Tag.

9. A DNA fragment encoding a polypeptide comprising the fusion polypeptide as defined in claim 6-8.

10. An expression cassette comprising a DNA fragment as defined in any of claims 5 and 9.

11. A cell which is capable of expressing a polypeptide and which is transformed with an expression cassette as defined in claim 10.

12. A process of producing a polypeptide, wherein a cell which contains a recombinant expression vector comprising a DNA fragment encoding a polypeptide according to claims 1-4, or a fusion polypeptide according to the claims 6-8 is cultured in a suitable medium under conditions which promote the expression of the polypeptide, and where the polypeptide is recovered from the culture.

13. Use of a polypeptide as defined in any of claims 1-4 for the screening of ligands binding to the polypeptide.

14. The use according to claim 13 for the screening for insulin mimetics.

## Patentansprüche

1. Polypeptid mit Bindungsaffinität für Insulin, wobei das Molekulargewicht des Polypeptids zwischen 40 kD und 100 kD liegt, mit der Formel
NH₂-A-B-COOH
wobei A eine Aminosäuresequenz ist, umfassend eine Sequenz korrespondierend zu SEQ ID NO: 1, SEQ ID NO: 2 oder SEQ ID NO: 3 oder ein funktionelles Äquivalent davon
und B eine Aminosäuresequenz ist, umfassend eine Sequenz korrespondierend zu SEQ ID NO: 4, SEQ ID NO: 5, SEQ ID NO: 6, SEQ ID NO: 7, SEQ ID NO: 8, SEQ
ID NO: 9, SEQ ID NO: 10 oder SEQ ID NO: 11 oder ein funktionelles Äquivalent davon, und wobei die Bindungsaffinität geringer ist als 10⁻⁸, bestimmt in einem Polyethylenglycol (PEG) Präzipitations-Assay.

2. Polypeptid nach Anspruch 1, wobei A eine Sequenz korrespondierend zu SEQ ID NO: 1, SEQ ID NO: 3 oder ein funktionelles Äquivalent davon und B eine Sequenz korrespondierend zu SEQ ID NO: 4, SEQ ID NO: 5, SEQ ID NO: 10 oder ein funktionelles Äquivalent davon, umfaßt.

3. Polypeptid nach einem der vorhergehenden Ansprüche, wobei das Molekulargewicht des Polypeptids unter 90 kDa, vorzugsweise unter 80 kDa, liegt.

4. Polypeptid nach einem der vorhergehenden Ansprüche, das höchstens 7, vorzugsweise höchstens 6 N-verknüpfte Glykosylierungsstellen aufweist.

5. DNA Fragment kodierend ein Polypeptid nach einem der Ansprüche 1 - 4.

6. Fusionspolypeptid umfassend ein Polypeptid nach einem der Ansprüche 1 - 5 und ein weiteres Polypeptid, wobei das weitere Polypeptid die Fähigkeit aufweist, die Expression, Reinigung und/oder Kristallisation des Polypeptids zu erleichtern.

7. Fusionspolypeptid nach Anspruch 6, umfassend ein antigenes Peptid-Tag und/oder FLAG Epitop.

8. Fusionspolypeptid nach Anspruch 6, umfassend ein polyHis und/oder 6His-Tag.

9. DNA Fragment kodierend ein Polypeptid umfassend das Fusionspolypeptid nach den Ansprüchen 6 - 8.

10. Expressionskassette umfassend ein DNA Fragment nach einem der Ansprüche 5 und 9.

11. Zelle, welche die Fähigkeit aufweist, ein Polypeptid zu exprimieren und die mit einer Expressionskassette nach Anspruch 10 transformiert ist.

12. Verfahren zur Herstellung eines Polypeptids, bei dem eine Zelle, die einen rekombinanten Expressionsvektor enthaltend ein DNA Fragment kodierend ein Polypeptid nach einem der Ansprüche 1 - 4 oder ein Fusionspolypeptid nach einem der Ansprüche 6 - 8, aufweist, in einem geeigneten Medium unter Bedingungen kultiviert wird, welche die Expression des Polypeptids unterstützen, und bei dem das Polypeptid aus der Kultur gewonnen wird.

13. Verwendung des Polypeptids nach einem der Ansprüche 1 - 4 zum Screenen von Liganden, die an das Polypeptid binden.

14. Verwendung nach Anspruch 13 zum Screenen von Insulin-nachahmenden Stoffen.

## Revendications

1. Polypeptide ayant une affinité de liaison pour l'insuline, dans lequel le poids moléculaire du polypeptide est compris entre 40 kD et 100 kD, ayant la formule suivante :
NH₂-A-B-COOH
où A est une séquence d'acides aminés comportant une séquence correspondant à la SEQ ID N° : 1, SEQ ID N° : 2 ou SEQ ID N° : 3 ou un équivalent fonctionnel de celles-ci,
et B est une séquence d'acides aminés comportant une séquence correspondant à la SEQ ID N° :4, SEQ ID N° : 5, SEQ ID N° : 6, SEQ ID N° : 7, SEQ ID N° : 8, SEQ ID N° : 9, SEQ ID N° : 10 ou SEQ ID N° : 11 ou un équivalent fonctionnel de celles-ci, et
dans lequel l'affinité de liaison est inférieure à 10^{- 8} telle que mesurée dans un essai de précipitation au polyéthylèneglycol (PEG) .

2. Polypeptide selon la revendication 1, dans lequel A comporte une séquence correspondant à la SEQ ID N° : 1, la SEQ ID N° : 3 ou un équivalent fonctionnel de celles-ci, et B comporte une séquence correspondant à la SEQ ID N° : 4, la SEQ ID N° : 5, la SEQ ID N° : 10 ou un équivalent fonctionnel de celles-ci.

3. Polypeptide selon l'une quelconque des revendications précédentes, dans lequel le poids moléculaire du polypeptide est inférieur à 90 kDa, de préférence inférieur à 80 kDa.

4. Polypeptide selon l'une quelconque des revendications précédentes, ayant au plus 7, de préférence au plus 6, sites de glycosylation attachés à N.

5. Fragment d'ADN codant pour un polypeptide tel que défini selon l'une quelconque des revendications 1 à 4.

6. Polypeptide de fusion comportant un polypeptide tel que défini selon l'une quelconque des revendications 1 à 5, et un polypeptide supplémentaire, dans lequel ledit polypeptide supplémentaire est capable de faciliter l'expression, la purification et/ou la cristallisation du polypeptide.

7. Polypeptide de fusion selon la revendication 6, comportant un peptide-Tag antigénique et/ou un épitope FLAG.

8. Polypeptide de fusion selon la revendication 6, comportant polyHis et/ou Tag 6His.

9. Fragment d'ADN codant pour un polypeptide comportant le polypeptide de fusion tel que défini selon les revendications 6 à 8.

10. Cassette d'expression comportant un fragment d'ADN tel que défini selon l'une quelconque des revendications 5 et 9.

11. Cellule qui est capable d'exprimer un polypeptide et qui est transformée par une cassette d'expression telle que définie dans la revendication 10.

12. Procédé de production d'un polypeptide, dans lequel une cellule qui contient un vecteur d'expression recombinant comportant un fragment d'ADN codant pour un polypeptide selon les revendications 1 à 4, ou un polypeptide de fusion selon les revendications 6 à 8, est cultivée dans un milieu adapté sous des conditions qui favorisent l'expression du polypeptide, et dans lequel le polypeptide est récupéré à partir de la culture.

13. Utilisation d'un peptide tel que défini selon l'une quelconque des revendications 1 à 4, pour le criblage de ligands se liant au polypeptide.

14. Utilisation selon la revendication 13, pour le criblage d'imitations d'insuline.


### (1) GENERAL INFORMATION:

(i) APPLICANT:
   (A) NAME: Novo Nordisk A/S
   (B) STREET: Novo Alle
   (C) CITY: Bagsvaerd
   (E) COUNTRY: Denmark
   (F) POSTAL CODE (ZIP): 2880
(ii) TITLE OF INVENTION: Insulin binding peptide
(iii) NUMBER OF SEQUENCES: 11
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: Patent In Release #1.0, Version #1.30 (EPO)

### (2) INFORMATION FOR SEQ ID NO: 1:

(i) SEQUENCE CHARACTERISTICS:
   (A) LENGTH: 468 amino acids
   (B) TYPE: amino acid
   (C) STRANDEDNESS:
   (D) TOPOLOGY: linear
(ii) MOLECULE TYPE: peptide
(v) FRAGMENT TYPE: internal
(xi) SEQUENCE DESCRIPTION: SEQ ID NO: 1:
   His Leu Tyr Pro Gly Glu Val Cys Pro Gly Met Asp Ile Arg Asn Asn
   1               5                   10                  15
   Leu Thr Arg Leu His Glu Leu Glu Asn Cys Ser Val Ile Glu Gly His
               20                  25                  30
   Leu Gln Ile Leu Leu Met Phe Lys Thr Arg Pro Glu Asp Phe Arg Asp
           35                  40                  45
   Leu Ser Phe Pro Lys Leu Ile Met Ile Thr Asp Tyr Leu Leu Leu Phe
       50                  55                  60
   Arg Val Tyr Gly Leu Glu Ser Leu Lys Asp Leu Phe Pro Asn Leu Thr
   65                  70                  75                  80
   Val Ile Arg Gly Ser Arg Leu Phe Phe Asn Tyr Ala Leu Val Ile Phe
                   85                  90                  95
   Glu Met Val His Leu Lys Glu Leu Gly Leu Tyr Asn Leu Met Asn Ile
                       100                 105                 110
   Thr Arg Gly Ser Val Arg Ile Glu Lys Asn Asn Glu Leu Cys Tyr Leu
               115                 120                 125
   Ala Thr Ile Asp Trp Ser Arg Ile Leu Asp Ser Val Glu Asp Asn Tyr
       130                 135                 140
   Ile Val Leu Asn Lys Asp Asp Asn Glu Glu Cys Gly Asp Ile Cys Pro
   145                 150                 155                 160
   Gly Thr Ala Lys Gly Lys Thr Asn Cys Pro Ala Thr Val Ile Asn Gly
                   165                 170                 175
   Gln Phe Val Glu Arg Cys Trp Thr His Ser His Cys Gln Lys Val Cys
               180                 185                 190
   Pro Thr Ile Cys Lys Ser His Gly Cys Thr Ala Glu Gly Leu Cys Cys
           195                 200                 205
   His Ser Glu Cys Leu Gly Asn Cys Ser Gln Pro Asp Asp Pro Thr Lys
       210                 215                 220
   Cys Val Ala Cys Arg Asn Phe Tyr Leu Asp Gly Arg Cys Val Glu Thr
   225                 230                 235                 240
   Cys Pro Pro Pro Tyr Tyr His Phe Gln Asp Trp Arg Cys Val Asn Phe
                   245                 250                 255
   Ser Phe Cys Gln Asp Leu His His Lys Cys Lys Asn Ser Arg Arg Gln
               260                 265                 270
   Gly Cys His Gln Tyr Val Ile His Asn Asn Lys Cys Ile Pro Glu Cys
           275                 280                 285
   Pro Ser Gly Tyr Thr Met Asn Ser Ser Asn Leu Leu Cys Thr Pro Cys
       290                 295                 300
   Leu Gly Pro Cys Pro Lys Val Cys His Leu Leu Glu Gly Glu Lys Thr
   305                 310                 315                 320
   Ile Asp Ser Val Thr Ser Ala Gln Glu Leu Arg Gly Cys Thr Val Ile
                   325                 330                 335
   Asn Gly Ser Leu Ile Ile Asn Ile Arg Gly Gly Asn Asn Leu Ala Ala
               340                 345                 350
   Glu Leu Glu Ala Asn Leu Gly Leu Ile Glu Glu Ile Ser Gly Tyr Leu
           355                 360                 365
   Lys Ile Arg Arg Ser Tyr Ala Leu Val Ser Leu Ser Phe Phe Arg Lys
       370                 375                 380
   Leu Arg Leu Ile Arg Gly Glu Thr Leu Glu Ile Gly Asn Tyr Ser Phe
   385                 390                 395                 400
   Tyr Ala Leu Asp Asn Gln Asn Leu Arg Gln Leu Trp Asp Trp Ser Lys
                   405                 410                 415
   His Asn Leu Thr Ile Thr Gln Gly Lys Leu Phe Phe His Tyr Asn Pro
   420 425 430
   Lys Leu Cys Leu Ser Glu Ile His Lys Met Glu Glu Val Ser Gly Thr
   435 440 445
   Lys Gly Arg Gln Glu Arg Asn Asp Ile Ala Leu Lys Thr Asn Gly Asp
   450 455 460
   Gln Ala Ser Cys
   465

### (2) INFORMATION FOR SEQ ID NO: 2:

(i) SEQUENCE CHARACTERISTICS:
   (A) LENGTH: 457 amino acids
   (B) TYPE: amino acid
   (C) STRANDEDNESS:
   (D) TOPOLOGY: linear
(ii) MOLECULE TYPE: peptide
(iv) ANTI-SENSE: NO
(v) FRAGMENT TYPE: internal
(xi) SEQUENCE DESCRIPTION: SEQ ID NO: 2:
   Glu Ile Cys Gly Pro Gly Ile Asp Ile Arg Asn Asp Tyr Gln Gln Leu
   1 5 10 15
   Lys Arg Leu Glu Asn Cys Thr Val Ile Glu Gly Tyr Leu His Ile Leu
   20 25 30
   Leu Ile Ser Lys Ala Glu Asp Tyr Arg Ser Tyr Arg Phe Pro Lys Leu
   35 40 45
   Thr Val Ile Thr Glu Tyr Leu Leu Leu Phe Arg Val Ala Gly Leu Glu
   50 55 60
   Ser Leu Gly Asp Leu Phe Pro Asn Leu Thr Val Ile Arg Gly Trp Lys
   65 70 75 80
   Leu Phe Tyr Asn Tyr Ala Leu Val Ile Phe Glu Met Thr Asn Leu Lys
   85 90 95
   Asp Ile Gly Leu Tyr Asn Leu Arg Asn Ile Thr Arg Gly Ala Ile Arg
   100 105 110
   Ile Glu Lys Asn Ala Asp Leu Cys Tyr Leu Ser Thr Val Asp Trp Ser
   115 120 125
   Leu Ile Leu Asp Ala Val Ser Asn Asn Tyr Ile Val Gly Asn Lys Pro
   130 135 140
   Pro Lys Glu Cys Gly Asp Leu Cys Pro Gly Thr Met Glu Glu Lys Pro
   145 150 155 160
   Met Cys Glu Lys Thr Thr Ile Asn Asn Glu Tyr Asn Tyr Arg Cys Trp
   165 170 175
   Thr Thr Asn Arg Cys Gln Lys Met Cys Pro Ser Thr Cys Gly Lys Arg
   180 185 190
   Ala Cys Thr Glu Asn Asn Glu Cys Cys His Pro Glu Cys Leu Gly Ser
   195 200 205
   Cys Ser Ala Pro Asp Asn Asp Thr Ala Cys Val Ala Cys Arg His Tyr
   210 215 220
   Tyr Tyr Ala Gly Val Cys Val Pro Ala Cys Pro Pro Asn Thr Tyr Arg
   225 230 235 240
   Phe Glu Gly Trp Arg Cys Val Asp Arg Asp Phe Cys Ala Asn Ile Leu
   245 250 255
   Ser Ala Glu Ser Ser Asp Ser Glu Gly Phe Val Ile His Asp Gly Glu
   260 265 270
   Cys Met Gln Glu Cys Pro Ser Gly Phe Ile Arg Asn Gly Ser Gln Ser
   275 280 285
   Met Tyr Cys Ile Pro Cys Glu Gly Pro Cys Pro Lys Val Cys Glu Glu
   290 295 300
   Glu Lys Lys Thr Lys Thr Ile Asp Ser Val Thr Ser Ala Gln Met Leu
   305 310 315 320
   Gln Gly Cys Thr Ile Phe Lys Gly Asn Leu Leu Ile Asn Ile Arg Arg
   325 330 335
   Gly Asn Asn Ile Ala Ser Glu Leu Glu Asn Phe Met Gly Leu Ile Glu
   340 345 350
   Val Val Thr Gly Tyr Val Lys Ile Arg His Ser His Ala Leu Val Ser
   355 360 365
   Leu Ser Phe Leu Lys Asn Leu Arg Leu Ile Leu Gly Glu Glu Gln Leu
   370 375 380
   Glu Gly Asn Tyr Ser Phe Tyr Val Leu Asp Asn Gln Asn Leu Gln Gln
   385 390 395 400
   Leu Trp Asp Trp Asp His Arg Asn Leu Thr Ile Lys Ala Gly Lys Met
   405 410 415
   Tyr Phe Ala Phe Asn Pro Lys Leu Cys Val Ser Glu Ile Tyr Arg Met
   420 425 430
   Glu Glu Val Thr Gly Thr Lys Gly Arg Ser Lys Gly Asp Ile Asn Thr
   435 440 445
   Arg Asn Asn Gly Glu Arg Ala Ser Cys
   450 455

### (2) INFORMATION FOR SEQ ID NO: 3:

(i) SEQUENCE CHARACTERISTICS:
   (A) LENGTH: 464 amino acids
   (B) TYPE: amino acid
   (C) STRANDEDNESS:
   (D) TOPOLOGY: linear
(ii) MOLECULE TYPE: peptide
(v) FRAGMENT TYPE: internal
(xi) SEQUENCE DESCRIPTION: SEQ ID NO: 3:
   His Leu Tyr Pro Gly Glu Val Cys Pro Gly Met Asp Ile Arg Asn Asn
   1               5                   10                  15
   Leu Thr Arg Leu His Glu Leu Glu Asn Cys Ser Val Ile Glu Gly His
               20                  25                  30
   Leu Gln Ile Leu Leu Met Phe Lys Thr Arg Pro Glu Asp Phe Arg Asp
           35                  40                  45
   Leu Ser Phe Pro Lys Leu Ile Met Ile Thr Asp Tyr Leu Leu Leu Phe
       50                  55                  60
   Arg Val Tyr Gly Leu Glu Ser Leu Gly Asp Leu Phe Pro Asn Leu Thr
   65                  70                  75                  80
   Val Ile Arg Gly Trp Lys Leu Phe Tyr Asn Tyr Ala Leu Val Ile Phe
                   85                  90                  95
   Glu Met Thr Asn Leu Lys Asp Ile Gly Leu Tyr Asn Leu Arg Asn Ile
               100                 105                 110
   Thr Arg Gly Ala Ile Arg Ile Glu Lys Asn Ala Asp Leu Cys Tyr Leu
           115                 120                 125
   Ser Thr Val Asp Trp Ser Leu Ile Leu Asp Ala Val Ser Asn Asn Tyr
       130                 135                 140
   Ile Val Gly Asn Lys Pro Pro Lys Glu Cys Gly Asp Leu Cys Pro Gly
   145                 150                 155                 160
   Thr Met Glu Glu Lys Pro Met Cys Glu Lys Thr Thr Ile Asn Asn Glu
                   165                 170                 175
   Tyr Asn Tyr Arg Cys Trp Thr Thr Asn Arg Cys Gln Lys Met Cys Pro
               180                 185                 190
   Ser Thr Cys Gly Lys Arg Ala Cys Thr Glu Asn Asn Glu Cys Cys His
           195                 200                 205
   Pro Glu Cys Leu Gly Ser Cys Ser Ala Pro Asp Asn Asp Thr Ala Cys
       210                 215                 220
   Val Ala Cys Arg His Tyr Tyr Tyr Ala Gly Val Cys Val Pro Ala Cys
   225                 230                 235                 240
   Pro Pro Asn Thr Tyr Arg Phe Glu Gly Trp Arg Cys Val Asp Arg Asp
                   245                 250                 255
   Phe Cys Ala Asn Ile Leu Ser Ala Glu Ser Ser Asp Ser Glu Gly Phe
               260                 265                 270
   Val Ile His Asp Gly Glu Cys Met Gln Glu Cys Pro Ser Gly Phe Ile
           275                 280                 285
   Arg Asn Gly Ser Gln Ser Met Tyr Cys Ile Pro Cys Glu Gly Pro Cys
       290                 295                 300
   Pro Lys Val Cys Glu Glu Glu Lys Lys Thr Lys Thr Ile Asp Ser Val
   305                 310                 315                 320
   Thr Ser Ala Gln Met Leu Gln Gly Cys Thr Ile Phe Lys Gly Asn Leu
                   325                 330                 335
   Leu Ile Asn Ile Arg Arg Gly Asn Asn Ile Ala Ser Glu Leu Glu Asn
               340                 345                 350
   Phe Met Gly Leu Ile Glu Val Val Thr Gly Tyr Val Lys Ile Arg His
           355                 360                 365
   Ser His Ala Leu Val Ser Leu Ser Phe Leu Lys Asn Leu Arg Leu Ile
       370                 375                 380
   Leu Gly Glu Glu Gln Leu Glu Gly Asn Tyr Ser Phe Tyr Val Leu Asp
   385                 390                 395                 400
   Asn Gln Asn Leu Gln Gln Leu Trp Asp Trp Asp His Arg Asn Leu Thr
                   405                 410                 415
   Ile Lys Ala Gly Lys Met Tyr Phe Ala Phe Asn Pro Lys Leu Cys Val
               420                 425                 430
   Ser Glu Ile Tyr Arg Met Glu Glu Val Thr Gly Thr Lys Gly Arg Gln
           435                 440                 445
   Ser Lys Gly Asp Ile Asn Thr Arg Asn Asn Gly Glu Arg Ala Ser Cys
       450                 455                 460

### (2) INFORMATION FOR SEQ ID NO: 4:

(i) SEQUENCE CHARACTERISTICS:
   (A) LENGTH: 28 amino acids
   (B) TYPE: amino acid
   (C) STRANDEDNESS:
   (D) TOPOLOGY: linear
(ii) MOLECULE TYPE: peptide
(v) FRAGMENT TYPE: internal (xi) SEQUENCE DESCRIPTION: SEQ ID NO: 4:

### (2) INFORMATION FOR SEQ ID NO: 5:

(i) SEQUENCE CHARACTERISTICS:
   (A) LENGTH: 16 amino acids
   (B) TYPE: amino acid
   (C) STRANDEDNESS:
   (D) TOPOLOGY: linear
(ii) MOLECULE TYPE: peptide
(v) FRAGMENT TYPE: internal
(xi) SEQUENCE DESCRIPTION SEQ ID NO: 5:
   Thr Phe Glu Asp Tyr Leu His Asn Val Val Phe Val Pro Arg Pro Ser
   1 5 10 15

### (2) INFORMATION FOR SEQ ID NO: 6:

(i) SEQUENCE CHARACTERISTICS:
   (A) LENGTH: 46 amino acids
   (B) TYPE: amino acid
   (C) STRANDEDNESS:
   (D) TOPOLOGY: linear
(ii) MOLECULE TYPE: peptide
(v) FRAGMENT TYPE: internal
(xi) SEQUENCE DESCRITION SEQ ID NO: 6:
   Pro Lys Thr Asp Ser Gln Ile Leu Lys Glu Leu Glu Glu Ser Ser Phe
   1 5 10 15
   Arg Lys Thr Phe Glu Asp Tyr Leu His Asn Val Val Phe Val Pro Arg
   20 25 30
   Lys Thr Ser Ser Gly Thr Gly Ala Glu Asp Pro Arg Pro Ser
   35 40 45

### (2) INFORMATION FOR SEQ ID NO: 7:

(i) SEQUENCE CHARACTERISTICS:
   (A) LENGTH: 34 amino acids
   (B) TYPE: amino acid
   (C) STRANDEDNESS:
   (D) TOPOLOGY: linear
(ii) MOLECULE TYPE: peptide
(v) FRAGMENT TYPE: internal
(xi) SEQUENCE DESCRIPTION SEQ ID NO: 7:
   Pro Lys Thr Asp Ser Gln Ile Leu Lys Glu Leu Glu Glu Ser Ser Phe
   1               5                   10                  15
   Arg Lys Thr Phe Glu Asp Tyr Leu His Asn Val Val Phe Val Pro Arg
               20                  25                  30
   Pro Ser

### (2) INFORMATION FOR SEQ ID NO: 8:

(i) SEQUENCE CHARACTERISTICS:
   (A) LENGTH: 57 amino acids
   (B) TYPE: amino acid
   (C) STRANDEDNESS:
   (D) TOPOLOGY: linear
(ii) MOLECULE TYPE: peptide
(v) FRAGMENT TYPE: internal
(xi) SEQUENCE DESCRIPTION: SEQ ID NO: 8:
   Tyr Glu Asp Ser Ala Gly Glu Cys Cys Ser Cys Pro Lys Thr Asp Ser
   1               5                   10                  15
   Gln Ile Leu Lys Glu Leu Glu Glu Ser Ser Phe Arg Lys Thr Phe Glu
               20                  25                  30
   Asp Tyr Leu His Asn Val Val Phe Val Pro Arg Lys Thr Ser Ser Gly
           35                  40                  45
   Thr Gly Ala Glu Asp Pro Arg Pro Ser
       50                  55

### (2) INFORMATION FOR SEQ ID NO: 9:

(i) SEQUENCE CHARACTERISTICS:
   (A) LENGTH: 45 amino acids
   (B) TYPE: amino acid
   (C) STRANDEDNESS:
   (D) TOPOLOGY: linear
(ii) MOLECULE TYPE: peptide
(v) FRAGMENT TYPE: internal
(xi) SEQUENCE DESCRIPTION: SEQ ID NO: 9:
   Tyr Glu Asp Ser Ala Gly Glu Cys Cys Ser Cys Pro Lys Thr Asp Ser
   1               5                   10                  15
   Gln Ile Leu Lys Glu Leu Glu Glu Ser Ser Phe Arg Lys Thr Phe Glu
               20                  25                  30
   Asp Tyr Leu His Asn Val Val Phe Val Pro Arg Pro Ser
           35                  40                  45

### (2) INFORMATION FOR SEQ ID NO: 10:

(i) SEQUENCE CHARACTERISTICS:
   (A) LENGTH: 16 amino acids
   (B) TYPE: amino acid
   (C) STRANDEDNESS:
   (D) TOPOLOGY: linear
(ii) MOLECULE TYPE: peptide
(v) FRAGMENT TYPE: internal
(xi) SEQUENCE DESCRIPTION:SEQ ID NO: 10:
   Val Phe Glu Asn Phe Leu His Asn Ser Ile Phe Val Pro Arg Pro Glu
   1               5                   10                  15

### (2) INFORMATION FOR SEQ NO: 11:

(i) SEQUENCE CHARACTERISTICS:
   (A) LENGTH: 34 amino acids
   (B) TYPE: amino acid
   (C) STRANDEDNESS:
   (D) TOPOLOGY: linear
(ii) MOLECULE TYPE: peptide
(v) FRAGMENT TYPE: internal
(xi) SEQUENCE DESCRIPTION: SEQ ID NO: 11: